(19) Europäisches Patentamt
European Patent Office
Office européen des brevets

(11) Publication number: **0 094 436 B1**

(12) **EUROPEAN PATENT SPECIFICATION**

(45) Date of publication of patent specification: **30.03.88**

(51) Int. Cl.[4]: **C 07 D 303/36**

(21) Application number: **82903587.2**

(22) Date of filing: **19.11.82**

(86) International application number: **PCT/JP82/00443**

(87) International publication number: **WO 83/01776 26.05.83 Gazette 83/13**

# (54) N-GLYCIDYL-SUBSTITUTED AMIDE COMPOUNDS.

(30) Priority: **20.11.81 JP 185534/81**
**21.07.82 JP 125886/82**

(43) Date of publication of application: **23.11.83 Bulletin 83/47**

(45) Publication of the grant of the patent: **30.03.88 Bulletin 88/13**

(84) Designated Contracting States: **FR**

(58) References cited:
**FR-A-2 108 063**
**JP-A-47 007 398**
**JP-A-47 009 048**
**US-A-2 131 120**
**US-A-2 730 531**
**US-A-3 798 242**
**US-A-3 904 658**
**US-A-3 931 058**

**The file contains technical information submitted after the application was filed and not included in this specification**

(73) Proprietor: **MITSUI TOATSU CHEMICALS, INCORPORATED**
**2-5 Kasumigaseki 3-chome**
**Chiyoda-Ku Tokyo 100 (JP)**

(72) Inventor: **ITOH, Hiroshi**
**521, Kasamacho Totsuka-ku**
**Yokohama-shi Kanagawa 247 (JP)**
Inventor: **NITTA, Atsuhiko**
**634-1-154, Nobacho Kounan-ku**
**Yokohama-shi Kanagawa 233 (JP)**
Inventor: **TANAKA, Tomio**
**8-14-2, Aoto**
**Katsushika-ku Tokyo 125 (JP)**
Inventor: **TSUBOI, Kenji**
**4-5-45, Dai**
**Kamakura-shi Kanagawa 247 (JP)**
Inventor: **KAMIO, Hideo**
**728-5, Sogabessho Odawara-shi**
**Kanagawa 250-02 (JP)**

(74) Representative: **Peaucelle, Chantal et al**
**Cabinet Plasseraud 84, rue d'Amsterdam**
**F-75009 Paris (FR)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 094 436 B1

**Description**

Technical field

This invention relates to novel N-monoglycidyl-substituted, N,N-diglycidyl-substituted, or N,N,N',N'-tetraglycidyl-substituted compounds derived from aliphatic or aromatic mono- and diamide compounds.

Background art

Conventionally, the following three methods are known to be typical of the methods for the preparation of glycidyl compounds:

i) Glycidyl ethers

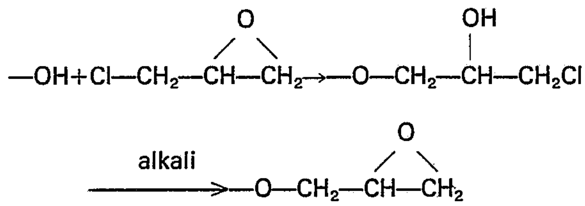


ii) Glycidylamines

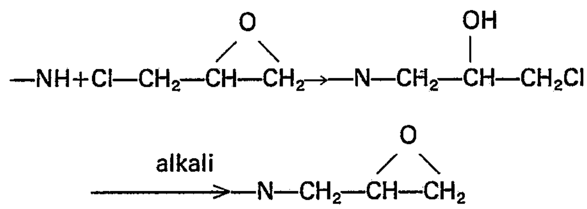


iii) Glycidyl esters

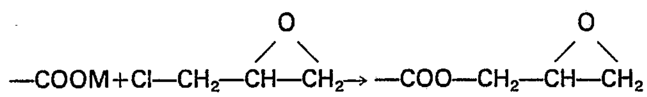

(M is an alkali metal ion.)

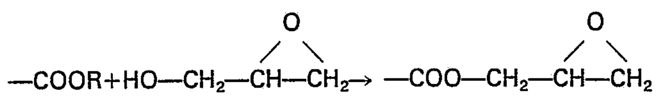

(R is a lower alkyl group.)

On the other hand, the following two methods are known to be typical of the methods for the preparation of N-substituted amide compounds:

(i) Method based on the reaction of an acid chloride with an amine

$$—COCl+NH_2R \rightarrow —CONHR$$

In order to prepare N-glycidyl-substituted amides according to this method, it is necessary to use glycidylamine of the formula

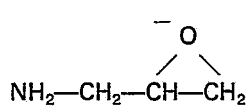

as a starting material. However, the use of this compound is impossible because it undergoes self-condensation and, hence, cannot exist in the free state.

(ii) Method based on the Ritter reaction

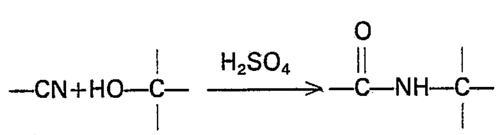


(tertiary alcohol)

Also in this method, it is impossible to use glycidyl alcohol of the formula

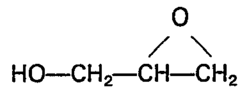

as a starting material, because it is a primary alcohol and undergoes ring cleavage under the action of an acid.

Thus, it has not been possible to prepare the N-glycidyl-substituted amide compounds provided by the present invention according to any conventional method.

Disclosure of the invention

It is an object of the present invention to provide novel N-glycidyl-substituted amide compounds which are useful in organic chemical reactions and as raw materials for the manufacture of organic high-molecular compounds such as reactive diluents, crosslinking agents, epoxidizing agents, resin modifiers, epoxy resins, adhesives, coating materials, electronic materials, composite materials, and the like.

The above object of the present invention is accomplished by providing N-glycidyl-substituted amide compounds and N-2-methylglycidyl-substituted amide compounds as described below.

Specifically, the present invention provides N-glycidyl-substituted amide compounds having the general formulas

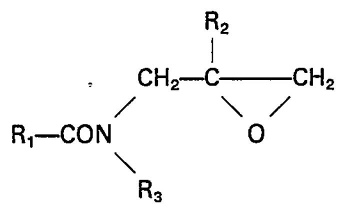

(I)

where

$R_1$ is an alkyl group having 1 or 2 carbon atoms, an alkenyl group having 2 or 3 carbon atoms, or an aryl group having 6 or 7 carbon atoms;

$R_2$ is a hydrogen atom or a methyl group; and

$R_3$ is an alkyl group having 1 to 4 carbon atoms or an alkenyl group having 3 to 6 carbon atoms, or an arylalkyl group having 1 to 3 carbon atoms in alkyl moiety,

provided that when $R_1$ is an alkyl group or an aryl group, $R_3$ is an alkenyl group; or

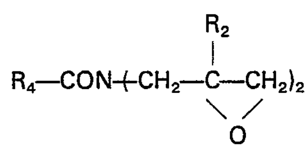

(II)

where

$R_2$ is a hydrogen atom or a methyl group; and

$R_4$ is an alkenyl group having 2 to 5 carbon atoms; or

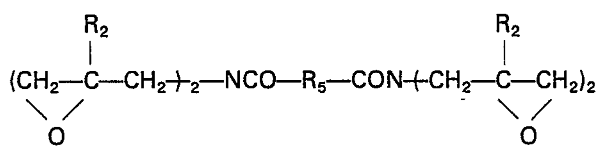

(III)

where

$R_2$ is a hydrogen atom or a methyl group; and

$R_5$ is a single bond, an alkylene group having 1 to 8 carbon atoms or an alkenylene group having 2 or 3 carbon atoms, or a phenylene group; or

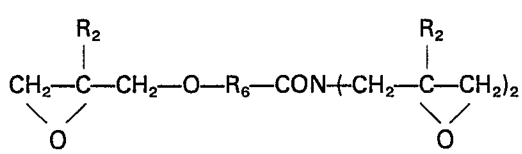

(IV)

where

$R_2$ is a hydrogen atom or a methyl group; and

$R_6$ is an alkylene group having 2 or 3 carbon atoms, or an arylene group having 6 or 7 carbon atoms.

The compounds which fall within the scope of the above general formulas are more specifically described below, although the description is limited to N-glycidyl-substituted compounds for purposes of simplification. First of all, they include N-monoglycidyl-substituted or N,N-diglycidyl-substituted compounds derived from aliphatic unsaturated monoamide compounds and the N-monoglycidyl-substituted or N,N-diglycidyl-substituted compounds derived from aromatic monoamide compounds.

As for polyamide compounds, N,N,N',N'-tetraglycidyl-substituted compounds derived from aliphatic saturated or unsaturated diamide compounds, and N,N,N',N'-tetraglycidyl-substituted compounds derived from aromatic diamide compounds are included.

Moreover, compounds obtained by introducing a glycidoxy group into N,N-diglycidyl-substituted compounds derived from aliphatic saturated monoamide compounds, compounds obtained by introducing a glycidoxy group into N,N-diglycidyl-substituted compounds derived from aromatic monoamide compounds are also included.

Best mode for carrying out the invention

In order to prepare the compounds of the present invention most efficiently, there can be employed the process for the preparation of N-substituted amide compounds which is disclosed in the previously filed FR Patent Application No. 8200623. More specifically, this process comprises reacting an amide compound with a halogen-substituted compound in an aprotic solvent in the presence of a strongly basic substance, whereby an N-substituted amide compound is prepared according to the following reaction formula:

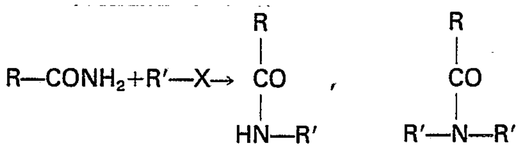

where R is, for example, an alkyl group, R' is an alkyl group, and X is a halogen.

In the above reaction formula, an epihalohydrin or dihalo-propanol, or a β-methylepihalohydrin or dihalo-β-methylpropanol may be used as the halogen-substituted compound to prepare N-substituted amides having a glycidyl or 2-methylglycidyl substituent, respectively. Moreover, a mixture of a compound as described above and a halide selected from alkyl halides, alkenyl halides, and aryl halides may be used as the halogen-substituted compound to prepare N,N-disubstituted amide compounds having a glycidyl or 2-methylglycidyl substituent and a residue corresponding to the halide.

Although the yield of the desired product is reduced as compared with the above-described process, the compounds of the present invention can also be prepared by a number of alternative processes. Specifically, they include a process in which an amide compound is reacted with a strongly basic substance in an aprotic solvent and, thereafter, a halogen-substituted compound is introduced into the reaction system to prepare an N-substituted amide compound; a process in which an amide compound is reacted with a halogen-substituted compound by the utilization of an phase transfer reaction to prepare an N-substituted amide compound; a process in which an N-substituted amide compound is prepared by using fluoride ions as a catalyst for the removal of hydrogen fluoride:

As regards N-glycidyl-substituted amides, for example, the compounds of the present invention are classified into a number of categories: N-glycidyl-substituted compounds derived from monoamide compounds, N-glycidyl-substituted compounds derived from diamide and N-glycidyl-substituted glycidyl ether compounds derived from hydroxyl-substituted monoamide compounds.

The N-glycidyl-substituted amide compounds derived from monoamide compounds include monoglycidyl-substituted compounds of the general formula (1)

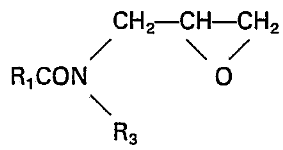

(1)

wherein

$R_1$ is selected from alkyl, alkenyl and aryl groups. The alkyl groups are represented by the general formula $C_nH_{2n+1}$— where n is an integer of 1 or 2. The alkenyl groups are represented by the general formula $C_nH_{2n-1}$— where n is an integer of 2 or 3. The aryl groups are substituent groups containing a benzene ring and are represented by the general formula $R_7$—$C_6H_4$— wherein $R_7$ is a hydrogen atom or a methyl group.

$R_3$ in the general formula (1) is selected from alkyl, alkenyl, and arylalkyl groups. The alkyl groups are represented by the general formula $C_nH_{2n+1}$— where n is an integer of 1 to 4. The alkenyl groups are represented by the general formula $C_nH_{2n-1}$— where n is an integer of 3 to 6. The arylalkyl groups are substituent groups containing a benzene ring and are represented by the general formula $C_6H_5$—$C_nH_{2n}$—, where n is an integer of 1 to 3, provided that when $R_1$ is an alkyl group or an aryl group, $R_3$ is an alkenyl group.

The N-glycidyl-substituted amide compounds derived from monoamide compounds further include diglycidyl-substituted compounds of the general formula (2)

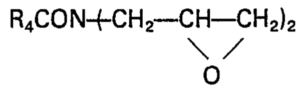 (2)

wherein $R_4$ is an alkenyl group represented by the general formula $C_nH_{2n-1}$, where n is an integer of 2 to 5.

Among the N-glycidyl-substituted amide compounds derived from polyamide compounds, those derived from diamide compounds can be represented by the general formula

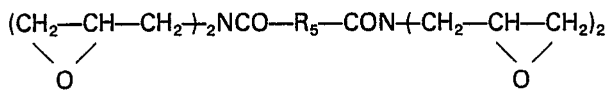 (3)

where $R_5$ is selected from a single bond, alkylene, alkenylene and phenylene groups. The alkylene groups are represented by the general formula $—C_nH_{2n}—$ where n is an integer of 1 to 8. The alkenylene groups are represented by the general formula $—C_nH_{2n-2}—$ where n is an integer of 2 or 3.

The N-glycidyl-substituted glycidyl ether compounds derived from hydroxyl-substituted monoamide compounds can be represented by the general formula

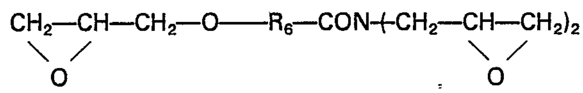 (4)

wherein $R_6$ is selected from alkylene and arylene groups.

The alkylene groups are represented by the general formula $—C_nH_{2n}—$ where n is an integer of 2 or 3. The arylene groups are substituent groups containing a benzene ring and are represented by the general formula

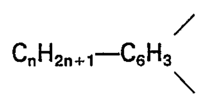

where n is an integer of 0 or 1.

Typical examples of the N-glycidyl-substituted amide compounds of the present invention are given hereinbelow. Among the N-glycidyl-substituted amide compounds derived from monoamide compounds, the N-monoglycidyl-substituted compounds include, for example, N-allyl-N-glycidylacetamide, N-methallyl-N-glycidylpropionamide, N-methyl-N-glycidylacrylamide, N-methyl-N-glycidylmethacrylamide, N-ethyl-N-glycidylcrotonamide, N-butyl-N-glycidylacrylamide, N-allyl-N-glycidylacrylamide, N-allyl-N-glycidylmethacrylamide, N-methallyl-N-glycidylcrotonamide, N-butenyl-N-glycidylmethacrylamide, N-benzyl-N-glycidylacrylamide, N-phenetyl-N-glycidylacrylamide, N-phenetyl-N-glycidylcrotonamide, N-phenyl-propyl-N-glycidylmethacrylamide, N-allyl-N-glycidylbenzamide, N-methallyl-N-glycidyltolylamide, N-hexenyl-N-glycidylallylbenzamide.

The N,N-diglycidyl-substituted compounds include, for example, N,N-diglycidylacrylamide, N,N-diglycidylmethacrylamide, N,N-diglycidylcrotonamide, N,N-diglycidyldivinylacetamide.

The N-glycidyl-substituted amide compounds derived from diamide compounds include, for example, N,N,N',N'-tetraglycidyloxamide, N,N,N',N'-tetraglycidylmalonamide, N,N,N',N'-tetraglycidylsuccinamide, N,N,N',N'-tetraglycidylglutaramide, N,N,N',N'-tetraglycidyladipamide, N,N,N',N'-tetraglycidylpimelamide, N,N,N',N'-tetraglycidylsuberamide, N,N,N',N'-tetraglycidylazelamide, N,N,N',N'-tetraglycidylsebacamide, N,N,N',N'-tetraglycidylfumaramide, N,N,N',N'-tetraglycidylmaleamide, N,N,N',N'-tetraglycidylcitraconamide, N,N,N',N'-tetraglycidylmeasaconamide, N,N,N',N'-tetraglycidylphthalamide, N,N,N',N'-tetraglycidylisophthalamide, N,N,N',N'-tetraglycidylterephthalamide.

On the other hand, the N-glycidyl-substituted glycidyl ether compounds derived from hydroxyl-substituted monoamide compounds include, for example, N,N-diglycidylglycidoxypropionamide, N,N-diglycidylglycidoxybutyramide, N,N-diglycidylglycidoxybenzamide, N,N-diglycidylglycidoxytolylamide.

Next, typical examples of the N-2-methylglycidyl-substituted compounds of the present invention are given hereinbelow. Among the N-2-methylglycidyl-substituted amide compound derived from monoamide compounds, the N-mono-2-methylglycidyl-substituted compounds include, for example, N-allyl-N-2-methylglycidylacetamide, N-methallyl-N-2-methylglycidylpropionamide, N-methyl-N-2-methylglycidylacrylamide, N-methyl-N-2-methylglycidylmethacrylamide, N-ethyl-N-2-methylglycidylcrotonamide, N-butyl-N-2-methylglycidylacrylamide, N-allyl-N-2-methylglycidylacrylamide, N-allyl-N-2-methylglycidylmethacrylamide, N-methallyl-N-2-methylglycidylcrotonamide, N-butenyl-N-2-methylglycidylmethacrylamide, N-benzyl-N-2-methylglycidylacrylamide, N-phenethyl-N-2-methylglycidylacrylamide, N-phenethyl-N-2-methylglycidylcrotonamide, N-phenylpropyl-N-2-methylglycidylmethacrylamide, N-hexenyl-N-2-methylglycidylallylbenzamide, N-benzyl-N-2-methylglycidylbenzamide, N-phenethyl-N-2-methylglycidyltolylamide.

The N,N-di-2-methylglycidyl-substituted compounds include, for example, N,N-di-2-methylglycidylacrylamide, N,N-di-2-methylglycidylmethacrylamide, N,N-di-2-methylglycidylcrotonamide, N,N-di-2-methylglycidyldivinylacetamide. The N-2-methylglycidyl-substituted amide compounds derived from diamide compounds include, for example, N,N,N',N'-tetra-2-methylglycidyloxamide, N,N,N',N'-tetra-2-methylglycidylmalonamide, N,N,N',N'-tetra-2-methylglycidylsuccinamide, N,N,N',N'-tetra-2-methylglycidylglutaramide, N,N,N',N'-tetra-2-methylglycidyladipamide, N,N,N',N'-tetra-2-methylglycidylpimelamide, N,N,N',N'-tetra-2-methylglycidylsuberamide, N,N,N',N'-tetra-2-methylglycidylazelamide, N,N,N',N'-tetra-2-methylglycidylsebacamide, N,N,N',N'-tetra-2-methylglycidylfumaramide, N,N,N',N'-tetra-2-methylglycidylmaleamide, N,N,N',N'-tetra-2-methylglycidylcitraconamide, N,N,N',N'-tetra-2-methylglycidylmesaconamide, N,N,N',N'-tetra-2-methylglycidylphthalamide, N,N,N',N'-tetra-2-methylglycidylisophthalamide, N,N,N',N'-tetra-2-methylglycidylterephthalamide.

On the other hand, the N-2-methylglycidyl-substituted 2-methylglycidyl ether compounds derived from hydroxyl-substituted monoamide compounds include, for example, N,N-di-2-methylglycidyl-2-methylglycidoxypropionamide, N,N-di-2-methylglycidyl-2-methylglycidoxybutyramide, N,N-di-2-methylglycidyl-2-methylglycidoxybenzamide, N,N-di-2-methylglycidyl-2-methylglycidoxytolylamide.

Among the above-enumerated compounds, N-glycidyl- or N-2-methylglycidyl-substituted compounds derived from lower fatty acid amides can be isolated and purified with comparative ease by such procedures as distillation and the like. However, other N-glycidyl- or N-2-methylglycidyl-substituted compounds, particularly N,N-diglycidyl or N,N-di-2-methylglycidyl compounds, are so low in volatility that it is difficult to isolate them by such procedures as distillation and the like. On the other hand, glycidyl and 2-methylglycidyl groups constitute highly reactive substituents and, therefore, react with reactive compounds such as alkaline substances coexisting in the reaction system to cause side reactions such as cleavage of the epoxy ring, the ensuing addition condensation. Accordingly, the epoxy equivalent of the product tends to become higher than the theoretical value and the magnitude of the deviation depends on the reactivity of the amide compound used. Generally, aromatic amide compounds seem to give an epoxy equivalent approaching the theoretical value.

The above-described N-glycidyl-substituted amide compounds and N-2-methylglycidyl-substituted amide compounds of the present invention have wide applications in the manufacture of reactive diluents, crosslinking agents, epoxidizing agents, resin modifiers, epoxy resins, adhesives, coating materials, electronic materials, composite materials, and the like, as is the case with conventionally known monoglycidyl ether compounds such as butyl glycidyl ether, allyl glycidyl ether, and phenyl glycidyl ether; monoglycidyl ester compounds such as glycidyl methacrylate, diglycidyl ether compounds typified by bisphenol epoxy resins; diglycidyl ester compounds such as diglycidyl phthalate; diglycidylamine compounds such as N,N-diglycidylaniline and N,N-diglycidyltoluidine; tetraglycidylamine compounds such as N,N,N',N'-tetraglycidylxylylenediamine and N,N,N',N'-tetraglycidyldiaminodiphenylmethane; mono-2-methylglycidyl ether compounds such as phenyl 2-methylglycidyl ether; mono-2-methylglycidyl ester compounds such as 2-methylglycidyl methacrylate; di-2-methylglycidyl ether compounds typified by methyl-substituted bisphenol epoxy resins; di-2-methylglycidyl ester compounds such as di-2-methylglycidyl terephthalate; di-2-methylglycidylamine compounds; tetra-2-methylglycidylamine compounds; and the like.

The present invention is further illustrated by the following examples.

Example 1 (Preparation of N,N-diglycidyl acrylamide)

To 150 ml of dimethyl sulfoxide (hereinafter abbreviated as DMSO) were added 14 g of acrylamide, 56 g of epichlorohydrin, 0.05 g of phenothiazine, and 20 g of sodium hydroxide. The resulting mixture was allowed to react at 40°C for 4 hours.

After completion of the reaction, the reaction mixture was filtered to remove any insoluble matter, and 200 ml of benzene and 150 ml of distilled water were added thereto. After vigorous stirring, the resulting mixture was separated in a separatory funnel and the aqueous layer was further extracted twice with 100-ml portions of benzene. The combined benzene layers were dried over magnesium sulfate and then distilled under reduced pressure. A fraction boiling at 118—120°C (3 mmHg) was collected to obtain 26 g of N,N-diglycidylacrylamide in a 72% yield.

Titration with perchlorate revealed that its epoxy equivalent was 93 g/eq. (theoretical value, 92 g/eq.).

Examples 2 and 3

Using the combinations of starting materials, strongly basic substance and solvent shown in Table 1, reaction was carried out under the conditions shown in Table 1. After completion of the reaction, the reaction mixture was worked up in entirely the same manner as in Example 1 to obtain the results shown in Table 2.

TABLE 1

| Example | Amide compound (g) | Halogen-substituted compound (g) | Strongly basic substance (g) | Solvent (ml) | Reaction temperature (°C)/time (h) |
|---|---|---|---|---|---|
| 2 | Methacrylamide, 17 | Epichlorohydrin, 56 | Sodium hydroxide, 20 | DMSO, 150 | 40/4 |
| 3 | Crotonamide, 17 | Epichlorohydrin, 56 | Potassium hydroxide, 28 | DMF, 150 | 40/3 |

DMF: N,N-Dimethylformamide.

TABLE 2

| Example | Product | Distillation conditions [temperature (°C)/ pressure (mmHg)] | Yield (g) (percentage) | Epoxy equivalent (g/eq.) (theoretical value) |
|---|---|---|---|---|
| 2 | N,N-diglycidyl-methacrylamide | 116—118/3 | 30(76) | 100(99) |
| 3 | N,N-Diglycidyl-crotonamide | 128—131/3 | 20(51) | 101(99) |

Example 4 (Preparation of N-n-butyl-N-glycidylacrylamide)

To 150 ml of DMSO were added 14 g of acrylamide, 37 g of epichlorohydrin, 46 g of n-butyl chloride, 20 g of sodium hydroxide, and 0.05 g of phenothiazine. The resulting mixture was allowed to react at 40°C for 5 hours.

After completion of the reaction, the reaction mixture was filtered to remove any insoluble matter, and then stripped of the starting materials and the solvent. To the residue were added 100 ml of benzene and 50 ml of distilled water. After vigorous stirring, the resulting mixture was separated in a separatory funnel and the aqueous layer was further extracted twice with 50-ml portions of benzene. The combined benzene layers were dried over magnesium sulfate and then distilled under reduced pressure. A fraction boiling at 103—105°C (5 mmHg) was collected to obtain 22 g of N-n-butyl-N-glycidylacrylamide in a 59% yield.

Titration with perchlorate revealed that its epoxy equivalent was 184 g/eq. (theoretical value, 183 g/eq.).

Examples 5 to 8

Using the combinations of starting materials, strongly basic substance and solvent shown in Table 3, reaction was carried out under the conditions shown in Table 3. After completion of the reaction, the reaction mixture was worked up in entirely the same manner as in Example 4 to obtain the results shown in Table 4.

TABLE 3

| Example | Amide compound (g) | Halogen-substituted compound (g) | Strongly basic substance (g) | Solvent (ml) | Reaction temperature (°C)/time (h) |
|---|---|---|---|---|---|
| 5 | Acrylamide, 14 | Allyl chloride, 23<br>Epichlorohydrin, 37 | Sodium hydroxide, 20 | DMSO, 150 | 40/4 |
| 6 | Acrylamide, 14 | Benzyl chloride, 38<br>Epichlorohydrin, 37 | Sodium hydroxide, 20 | DMF, 150 | 40/5 |
| 7 | Methacrylamide, 14 | Allyl chloride, 23<br>Epichlorohydrin, 37 | Sodium hydroxide, 20 | DMSO, 150 | 40/5 |
| 8 | Acetamide, 12 | Allyl chloride, 23<br>Epichlorohydrin, 37 | Sodium hydroxide, 20 | DMSO, 150 | 40/5 |

TABLE 4

| Example | Product | Distillation conditions [temperature (°C)/pressure (mmHg)] | Yield (g) (percentage) | Epoxy equivalent (g/eq.) (theoretical value) |
|---|---|---|---|---|
| 5 | N-Allyl-N-glycidyl-acrylamide | 74—76/2 | 21(62) | 168(167) |
| 6 | N-Benzyl-N-glycidyl-acrylamide | 137—139/2 | 22(51) | 218(217) |
| 7 | N-Allyl-N-glycidyl-methacrylamide | 82—84/3 | 23(63) | 181(181) |
| 8 | N-Allyl-N-glycidyl-acetamide | 76—78/3 | 22(70) | 155(155) |

Example 9 (Preparation of N,N,N',N'-tetraglycidylisophthalamide)

To 150 ml of DMSO were added 25 g of isophthalamide, 83 g of epichlorohydrin, and 28 g of sodium hydroxide. The resulting mixture was allowed to react at 40°C for 4 hours.

After completion of the reaction, the reaction mixture was filtered to remove any insoluble matter, and then stripped of the starting materials and the solvent. To the residue were added 100 ml of benzene and 50 ml of distilled water. After vigorous stirring, the resulting mixture was separated in a separatory funnel and the aqueous layer was further extracted twice with 50-ml portions of benzene. The combined benzene layers were dried over magnesium sulfate. After benzene was distilled off, the remaining solvent and the like were removed by distillation at 120°C and 2 mmHg to obtain 51 g of N,N,N',N'-tetraglycidylisophthalamide in a 88% yield.

Titration with perchlorate revealed that its epoxy equivalent was 107 g/eq. (theoretical value, 97 g/eq.). Moreover, its refractive index at 25°C was found to be 1.5469.

Examples 10 to 18

Using the combinations of starting materials, strongly basic substance and solvent shown in Table 5, reaction was carried out under the conditions shown in Table 5. After completion of the reaction, the reaction mixture was worked up in entirely the same manner as in Example 11 to obtain the results shown in Table 6.

TABLE 5

| Example | Amide compound (g) | Halogen-substituted compound (g) | Strongly basic substance (g) | Solvent (ml) | Reaction temperature (°C)/time (h) |
|---|---|---|---|---|---|
| 10 | Terephthalamide, 25 | Epichlorohydrin, 83 | Potassium hydroxide, 39 | DMSO, 150 | 50/4 |
| 11 | Phthalamide, 25 | Epichlorohydrin, 83 | Potassium hydroxide, 39 | DMSO, 150 | 50/4 |
| 12 | p-Hydroxy-benzamide, 27 | Epichlorohydrin, 74 | Sodium hydroxide, 28 | DMSO, 150 | 40/4 |
| 13 | Salicylamide, 27 | Epichlorohydrin, 74 | Potassium hydroxide, 39 | DMAC, 39 | 40/5 |
| 14 | Fumaramide, 17 | Epichlorohydrin, 83 | Sodium hydroxide, 28 | DMSO, 150 | 40/4 |
| 15 | Lactamide, 18 | Epichlorohydrin, 74 | Sodium hydroxide, 28 | DMSO, 150 | 40/4 |
| 16 | β-Hydroxy-propionamide, 18 | Epichlorohydrin, 74 | Sodium hydroxide, 28 | DMSO, 150 | 40/4 |
| 17 | Adipamide, 21 | Epichlorohydrin, 83 | Potassium hydroxide, 39 | DMSO, 150 | 50/4 |
| 18 | Oxamide, 13 | Epichlorohydrin, 83 | Potassium hydroxide, 39 | DMSO, 150 | 50/4 |

DMAC: N,N-Dimethylacetamide.

TABLE 6

| Example | Product | Yield (g) (percentage) | Refractive index (25°C) | Epoxy equivalent (g/eq.) (theoretical value) |
|---|---|---|---|---|
| 10 | N,N,N',N'-Tetraglycidylterephthalamide | 44(76) | 1.5378 | 113(97) |
| 11 | N,N,N',N'-Tetraglycidylphthalamide | 35(61) | 1.4950 | 131(97) |
| 12 | N,N-Diglycidyl-p-glycidoxybenzamide | 48(78) | 1.5525 | 111(102) |
| 13 | N,N-Diglycidyl-o-glycidoxybenzamide | 40(65) | 1.5493 | 132(102) |
| 14 | N,N,N',N'-Tetraglycidylfumaramide | 26(52) | 1.5002 | 100(85) |
| 15 | N,N-Diglycidyl-α-glycidoxypropionamide | 39(76) | 1.4842 | 117(86) |
| 16 | N,N-Diglycidyl-β-glycidoxypropionamide | 43(83) | 1.4796 | 91(86) |
| 17 | N,N,N',N'-Tetraglycidyladipamide | 5(8) | 1.5040 | 142(92) |
| 18 | N,N,N',N'-Tetraglycidyloxamide | 23(48) | 1.4863 | 109(78) |

Example 19 (Preparation of N-methyl-N-glycidylacrylamide)

To 150 ml of sulfolane were added 14 g of acrylamide, 0.05 g of phenothiazine, 37 g of epichlorohydrin, and 20 g of sodium hydroxide, followed by bubbling 15 g of methyl chloride thereinto. The resulting mixture was allowed to react at 40°C for 3 hours.

After completion of the reaction, the reaction mixture was filtered to remove any insoluble matter, and the filtrate was distilled under reduced pressure. A fraction boiling at 57—59°C (2 mmHg) was collected to obtain 18 g of N-methyl-N-glycidylacrylamide in a 63% yield.

Titration with perchlorate revealed that its epoxy equivalent was 142 g/eq. (theoretical value, 141 g/eq.).

Example 20

Using the combinations of starting materials, strongly basic substance and solvent shown in Table 7, reaction was carried out under the conditions shown in Table 7. After completion of the reaction, the reaction mixture was worked up in entirely the same manner as in Example 19 to obtain the results shown in Table 8.

TABLE 7

| Example | Amide compound (g) | Halogen-substituted compound (g) | Strongly basic substance (g) | Solvent (ml) | Reaction temperature (°C)/time (h) |
|---|---|---|---|---|---|
| 20 | Methacryl-amide, 17 | Methyl chloride, 15<br>Epichloro-hydrin, 37 | Potassium hydroxide, 28 | Tetraglyme, 150 | 40/2 |

TABLE 8

| Example | Product | Distillation conditions [temperature (°C)/ pressure (mmHg)] | Yield (g) (percentage) | Epoxy equiv-alent (g/eq.) (theoretical value) |
|---|---|---|---|---|
| 20 | N-Methyl-N-glycidyl-methacrylamide | 59—61/3 | 20(64) | 155(155) |

Example 21 (Adhesion test)

N,N-Diglycidyl-p-glycidoxybenzamide, which had been prepared in Example 12, was subjected to an adhesion test in which steel plates were bonded with the following formulation.

o Formulation

A mixture of 100 parts of N,N-diglycidyl-p-glycidoxybenzamide, 6 parts of dicyandiamide, and 2 parts of aerosil was intimately blended in a three-roll mill. Subsequently, 30 parts of alumina was uniformly dispersed in the blend, which was deaerated under reduced pressure to obtain a formulation.

o Preparation of a specimen

A 25 mm (width)×100 mm (length)×1.6 mm (thickness) steel plate (JIS G3141) was degreased with acetone, and the above formulation was applied to a terminal region of one surface thereof to a length of 12.5 mm. Another similar steel plate was superposed thereon and both plates were fastened with a clip. Then, the formulation was cured at 180°C for 60 minutes to form a specimen.

o Testing

When measured according to the procedure of JIS K6850, the tensile shear strength of the specimen was found to be 190 $kg/cm^2$.

Example 22 (Preparation of N,N-di-2-methylglycidylacrylamide)

To 250 ml of DMF were added 18 g of acrylamide, 133 g of β-methylepichlorohydrin, 0.05 g of phenothiazine, and 50 g of sodium hydroxide. The resulting mixture was allowed to react at 30°C for 5 hours.

After completion of the reaction, the reaction mixture was filtered to remove any insoluble matter, and then stripped of the starting materials and the solvent. To the residue were added 200 ml of benzene and 100 ml of distilled water. After vigorous stirring, the resulting mixture was separated in a separatory funnel and the aqueous layer was further extracted twice with 100-ml portions of benzene. The combined benzene layers were dried over magnesium sulfate and then distilled under reduced pressure. A fraction boiling at 95°C (0.3 mmHg) was collected to obtain 39 g of N,N-di-2-methylglycidylacrylamide in a 74% yield.

Titration with perchlorate revealed that its epoxy equivalent was 107 g/eq. (theoretical value, 106 g/eq.).

Example 23

Using the combinations of starting materials, strongly basic substance and solvent shown in Table 9 and the reaction conditions also shown in Table 9, reaction was carried out in entirely the same manner as in Example 22, except that phenothiazine was not added to the reaction mixture.

After completion of the reaction, the reaction mixture was worked up in entirely the same manner as in Example 22 to obtain the results shown in Table 10.

Example 24 (Preparation of N-allyl-N-2-methylglycidylacrylamide)

To 250 ml of DMF were added 18 g of acrylamide, 57 g of allyl chloride, 80 g of β-methylepichlorohydrin, 42 g of potassium hydroxide, and 0.05 g of phenothiazine. The resulting mixture was allowed to react at 30°C for 5 hours.

After completion of the reaction, the reaction mixture was filtered to remove any insoluble matter, and then stripped of the starting materials and the solvent. To the residue were added 200 ml of benzene and 100 ml of distilled water. After vigorous stirring, the resulting mixture was separated in a separatory funnel and the aqueous layer was further extracted twice with 100-ml portions of benzene. The combined benzene layers were dried over magnesium sulfate and then distilled under reduced pressure. A fraction boiling at 53—55°C (0.9 mmHg) was collected to obtain 24 g of N-allyl-N-2-methylglycidylacrylamide in a 53% yield.

Titration with perchlorate revealed that its epoxy equivalent was 182 g/eq. (theoretical value, 181 g/eq.).

Examples 25 to 27

Using the combinations of starting materials, strongly basic substance and solvent shown in Table 11 and the reaction conditions also shown in Table 11, reaction was carried out in entirely the same manner as in Example 24.

After completion of the reaction, the reaction mixture was worked up in entirely the same manner as in Example 24 to obtain the results shown in Table 12.

Example 28 (Preparation of N,N,N',N'-tetra-2-methylglycidylisophthalamide)

To 250 ml of DMSO were added 21 g of isophthalamide, 133 g of β-methylepichlorohydrin, and 30 g of sodium hydroxide. The resulting mixture was allowed to react at 30°C for 5 hours.

After completion of the reaction, the reaction mixture was filtered to remove any insoluble matter, and then stripped of the starting materials and the solvent. To the residue were added 200 ml of benzene and 100 ml of distilled water. After vigorous stirring, the resulting mixture was separated in a separatory funnel and the aqueous layer was further extracted twice with 100-ml portions of benzene. The combined benzene layers were dried over magnesium sulfate. After benzene was distilled off, the remaining solvent and the like were removed by distillation at 120°C and 2 mmHg to obtain 42 g of N,N,N',N'-tetra-2-methylglycidylisophthalamide in an 83% yield.

Titration with perchlorate revealed that its epoxy equivalent was 121 g/eq. (theoretical value, 111 g/eq.).

Moreover, its refractive index at 25°C was found to be 1.5311.

Examples 29 to 33

Using the combinations of starting materials, strongly basic substance and solvent shown in Table 13 and the reaction conditions also shown in Table 13, reaction was carried out in entirely the same manner as in Example 28.

After completion of the reaction, the reaction mixture was worked up in entirely the same manner as in Example 28 to obtain the results shown in Table 14.

Example 34

N,N-Di-2-methylglycidyl-p-2-methylglycidoxybenzamide, which had been prepared in Example 30, was subjected to an adhesion test in which steel plates were bonded with the following formulation.

o Formulation

A mixture of 100 parts of N,N-di-2-methylglycidyl-p-2-methylglycidoxybenzamide, 5 parts of dicyandiamide, and 2 parts of Aerosil® was intimately blended in a three-roll mill. Subsequently, 30 parts of alumina was uniformly dispersed in the blend, which was deaerated under reduced pressure to obtain a formulation.

o Preparation of a specimen

A 25 mm (width)×100 mm (length)×1.6 mm (thickness) steel plate (JIS G3141) was degreased with acetone, and the above formulation was applied to a terminal region of one surface thereof to a length of

12.5 mm. Another similar steel plate was superposed thereon and both plates were fastened with a clip. Then, the formulation was cured at 180°C for 60 minutes to form a specimen.

o Testing

When measured according to the procedure of JIS K6850, the tensile shear strength of the specimen was found to be 180 kg/cm$^2$.

TABLE 9

| Example | Amide compound (g) | Halogen-substituted compound (g) | Strongly basic substance (g) | Solvent (ml) | Reaction temperature (°C)/time (h) |
|---|---|---|---|---|---|
| 23 | Methacrylamide, 22 | β-Methylepichlorohydrin, 133 | Sodium hydroxide, 30 | DMF, 250 | 30/5 |

TABLE 10

| Example | Product | Distillation conditions [temperature (°C)/ pressure (mmHg)] | Yield (g) (percentage) | Epoxy equivalent (g/eq.) (theoretical value) |
|---|---|---|---|---|
| 23 | N,N-Di-2-methylglycidylmethacrylamide | 87/0.3 | 41(73) | 114(113) |

TABLE 11

| Example | Amide compound (g) | Halogen-substituted compound (g) | Strongly basic substance (g) | Solvent (ml) | Reaction temperature (°C)/time (h) |
|---|---|---|---|---|---|
| 25 | Methacrylamide, 22 | Allyl chloride, 57<br>β-Methylepichlorohydrin, 80 | Potassium hydroxide, 42 | DMF, 250 | 30/5 |
| 26 | Acetamide, 15 | Allyl chloride, 57<br>β-Methylepichlorohydrin, 80 | Potassium hydroxide, 42 | DMF, 250 | 30/5 |
| 27 | Acrylamide 18 | 1-Bromobutane, 48<br>β-Methylepichlorohydrin, 80 | Potassium hydroxide, 42 | DMSO, 250 | 30/5 |

TABLE 12

| Example | Product | Distillation condition [temperature (°C)/ pressure (mmHg)] | Yield (g) (percentage) | Epoxy equivalent (g/eq.) (theoretical value) |
|---|---|---|---|---|
| 25 | N-Allyl-N-2-methylglycidylmethacrylamide | 62/0.3 | 25(51) | 196(195) |
| 26 | N-Allyl-N-2-methylglycidylacetamide | 40/0.4 | 17(41) | 170(169) |
| 27 | N-Butyl-N-2-methylglycidylacrylamide | 34/0.3 | 25(51) | 199(197) |

TABLE 13

| Example | Amide compound (g) | Halogen-substituted compound (g) | Strongly basic substance (g) | Solvent (ml) | Reaction temperature (°C)/time (h) |
|---|---|---|---|---|---|
| 29 | Terephthalamide, 21 | β-Methylepichlorohydrin, 133 | Sodium hydroxide, 30 | DMSO, 250 | 50/5 |
| 30 | p-Hydroxybenzamide, 26 | β-Methylepichlorohydrin, 133 | Sodium hydroxide, 30 | DMSO, 250 | 20/5 |
| 31 | Salicylamide, 26 | β-Methylepichlorohydrin, 133 | Sodium hydroxide, 30 | DMSO, 250 | 20/5 |
| 32 | Oxamide, 11 | β-Methylepichlorohydrin, 133 | Sodium hydroxide, 30 | DMSO, 250 | 30/5 |
| 33 | Lactamide, 17 | β-Methylepichlorohydrin, 133 | Sodium hydroxide, 30 | DMSO, 250 | 20/5 |

TABLE 14

| Example | Product | Yield (g) (percentage) | Refractive index (25°C) | Epoxy equivalent (g/eq.) (theoretical value) |
|---|---|---|---|---|
| 29 | N,N,N',N'-Tetra-2-methylglycidylterephthalamide | 34(61) | 1.5262 | 140(111) |
| 30 | N,N-Di-2-methylglycidyl-p-2-methylglycidoxybenzamide | 49(74) | 1.5345 | 150(116) |
| 31 | N,N-Di-2-methylglycidyl-o-2-methylglycidoxybenzamide | 31(47) | 1.5498 | 208(116) |
| 32 | N,N,N',N'-Tetra-2-methylglycidyloxamide | 21(45) | 1.4869 | 106(92) |
| 33 | N,N-Di-2-methylglycidyl-α-2-methylglycidoxypropionamide | 33(58) | 1.4705 | 133(100) |

**Claims**

1. An N-glycidyl-substituted amide compound having the general formula (I)

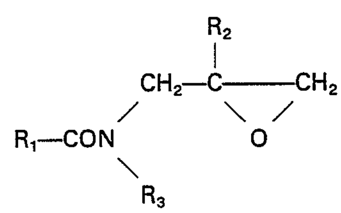

(I)

where

$R_1$ is an alkyl group having 1 or 2 carbon atoms, an alkenyl group having 2 or 3 carbon atoms, or an aryl group having 6 or 7 carbon atoms;

$R_2$ is a hydrogen atom or a methyl group; and

$R_3$ is an alkyl group having 1 to 4 carbon atoms, an alkenyl group having 3 to 6 carbon atoms, or an arylalkyl group having 1 to 3 carbon atoms in alkyl moiety, provided that when $R_1$ is an alkyl group or an aryl group, $R_3$ is an alkenyl group.

2. An N-glycidyl-substituted amide compound having the general formula (II)

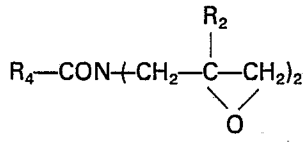

(II)

where
$R_2$ is a hydrogen atom or a methyl group; and
$R_4$ is an alkenyl group having 2 to 5 carbon atoms.

3. An N-glycidyl-substituted amide compound having the general formula (III)

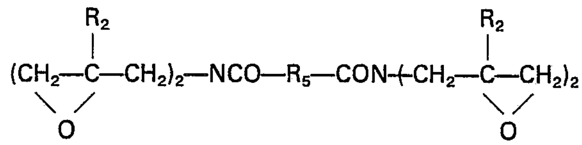

(III)

where
$R_2$ is a hydrogen atom or a methyl group; and
$R_5$ is a single bond, an alkylene group having 1 to 8 carbon atoms or an alkenylene group having 2 or 3 carbon atoms, or a phenylene group.

4. An N-glycidyl-substituted amide compound having the general formula (IV)

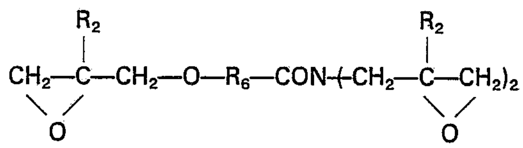

(IV)

where
$R_2$ is a hydrogen atom or a methyl group; and
$R_6$ is an alkylene group having 2 or 3 carbon atoms, or an arylene group having 6 or 7 carbon atoms.

5. A compound selected from N,N-diglycidylacrylamide, N,N-diglycidylmethacrylamide, N,N-diglycidylcrotonamide, N-methyl-N-glycidylacrylamide, N-methyl-N-glycidylmethacrylamide, N-n-butyl-N-glycidylacrylamide, N-allyl-N-glycidylacetamide, N-allyl-N-glycidylacrylamide, N-benzyl-N-glycidylacrylamide or N-allyl-N-glycidylmethacrylamide.

6. A compound selected from N,N-di-2-methylglycidylacrylamide, N,N-di-2-methylglycidylmethacrylamide, N-butyl-N-2-methylglycidylacrylamide, N-allyl-N-2-methylglycidylacetamide, N-allyl-N-2-methylglycidylacrylamide or N-allyl-N-2-methylglycidylmethacrylamide.

7. A compound selected from N,N,N',N'-tetraglycidyloxamide, N,N,N',N'-tetraglycidyladipamide, N,N,N',N'-tetraglycidylfumaramide, N,N,N',N'-tetraglycidylisophthalamide, N,N,N',N'-tetraglycidylterephthalamide or N,N,N',N'-tetraglycidylphtalamide.

8. A compound selected from N,N,N',N'-tetra-2-methylglycidyloxamide, N,N,N',N'-tetra-2-méthylglycidylisophthalamide or N,N,N',N'-tetra-2-methylglycidylterephthalamide.

9. A compound selected from N,N,diglycidyl-α-glycidoxypropionamide, N,N-diglycidyl-β-glycidoxypropionamide, N,N-diglycidyl-p-glycidoxybenzamide or N,N-diglycidyl-o-glycidoxybenzamide.

10. A compound selected from N,N-di-2-methylglycidyl-α-2-methylglycidoxypropionamide, N,N-di-2-methylglycidyl-p-2-methylglycidoxybenzamide or N,N-di-2-methylglycidyl-o-2-methylglycidoxybenzamide.

**Patentansprüche**

1. N-glycidyl-substituierte Amidverbindung der allgemeinen Formel (I)

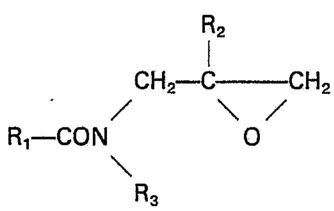

(I)

in der
$R_1$ eine Alkylgruppe mit 1 oder 2 Kohlenstoffatomen, eine Alkenylgruppe mit 2 oder 3 Kohlenstoffatomen, oder eine Arylgruppe mit 6 oder 7 Kohlenstoffatomen ist;

$R_2$ ein Wasserstoffatom oder eine Methylgruppe ist; und

$R_3$ eine Alkylgruppe mit 1 bis 4 Kohlenstoffatomen, eine Alkenylgruppe mit 3 bis 6 Kohlenstoffatomen, oder eine Arylalkylgruppe mit 1 bis 3 Kohlenstoffatomen in der Alkyleinheit mit der Massgabe ist, dass $R_3$ eine Alkenylgruppe ist, wenn $R_1$ eine Alkylgruppe oder eine Arylgruppe ist.

2. N-glycidyl-substituierte Amidverbindung der allgemeinen Formel (II)

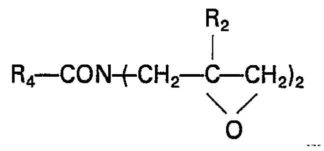

(II)

in der

$R_2$ ein Wasserstoffatom oder eine Methylgruppe ist; und

$R_4$ eine Alkenylgruppe mit 2 bis 5 Kohlenstoffatomen ist.

3. N-glycidyl-substituierte Amidverbindung der allgemeinen Formel (III)

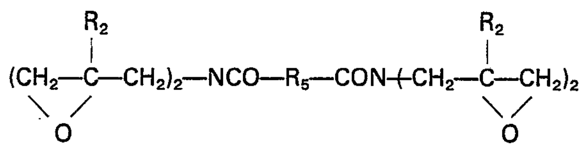

(III)

in der

$R_2$ ein Wasserstoffatom oder eine Methylgruppe ist; und

$R_5$ eine Einfachbindung, eine Alkylengruppe mit 1 bis 8 Kohlenstoffatomen, oder eine Alkenylengruppe mit 2 oder 3 Kohlenstoffatomen oder eine Phenylengruppe ist.

4. N-glycidyl-substituierte Amidverbindung der allgemeinen Formel (IV)

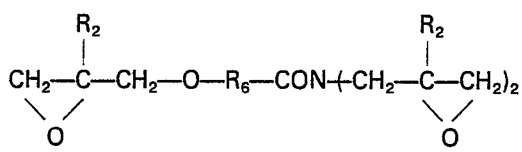

(IV)

in der

$R_2$ ein Wasserstoffatom oder eine Methylgruppe ist; und

$R_6$ eine Alkylengruppe mit 2 oder 3 Kohlenstoffatomen oder eine Arylengruppe mit 6 oder 7 Kohlenstoffatomen ist.

5. Verbindung ausgewählt aus N,N-Diglycidylacrylamid, N,N-Diglycidylmethacrylamid, N,N-Diglycidylcrotonamid, N-Methyl-N-glycidylacrylamid, N-Methyl-N-glycidylmethacrylamid, N-n-Butyl-N-glycidylacrylamid, N-Allyl-N-glycidylacetamid, N-Allyl-N-glycidylacrylamid, N-Benzyl-N-glycidylacrylamid, oder N-Allyl-N-glycidylmethacrylamid.

6. Verbindung ausgewählt aus N,N-di-2-Methylglycidylacrylamid, N,N-di-2-Methylglycidylmethacrylamid, N-Butyl-N-2-methylglycidylacrylamid, N-Allyl-N-2-methylglycidylacetamid, N-Allyl-N-2-methylglycidylacrylamid oder N-Allyl-N-2-methylglycidylmethacrylamid.

7. Verbindung ausgewählt aus N,N,N',N'-Tetraglycidyloxamid, N,N,N',N'-Tetraglycidyladipamid, N,N,N',N'-Tetraglycidylfumaramid, N,N,N',N'-Tetraglycidylisophthalamid, N,N,N',N'-Tetraglycidylterephthalamid oder N,N,N',N'-Tetraglycidylphtalamid.

8. Verbindung ausgewählt aus N,N,N',N'-tetra-2-Methylglycidyloxamid, N,N,N',N'-tetra-2-Methylglycidylisophthalamid oder N,N,N',N'-tetra-2-Methylglycidylterephthalamid.

9. Verbindung ausgewählt aus N,N-Diglycidyl-α-glycidoxypropionamid, N,N-Diglycidyl-β-glycidoxypropionamid, N,N-Diglycidyl-p-glycidoxybenzamid oder N,N-Diglycidyl-o-glycidoxybenzamid.

10. Verbindung ausgewählt aus N,N-di-2-Methylglycidyl-α-2-methylglycidoxypropionamid, N,N-di-2-Methylglycidyl-p-2-methylglycidoxybenzamid oder N,N-di-2-Methylglycidyl-o-2-methylglycidoxybenzamid.

**Revendications**

1. Composé amidé N-glycidyl-substitué ayant la formule générale (I)

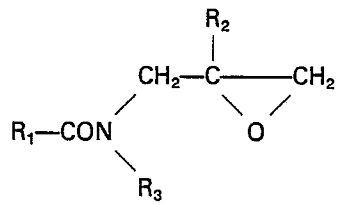

(I)

dans laquelle

$R_1$ est un groupe alcoyle ayant 1 ou 2 atomes de carbone, un groupe alcényle ayant 2 ou 3 atomes de carbone, ou un groupe aryle ayant 6 ou 7 atomes de carbone;

$R_2$ est un atome d'hydrogène ou un groupe méthyle; et

$R_3$ est un groupe alcoyle ayant 1 à 4 atomes de carbone, un groupe alcényle ayant 3 à 6 atomes de carbone, ou un groupe arylalcoyle ayant 1 à 3 atomes de carbone dans la partie alcoyle, étant entendu que lorsque $R_1$ est un groupe alcoyle ou un groupe aryle, $R_3$ est un groupe alcényle.

2. Composé amidé N-glycidyl-substitué ayant la formule générale (II)

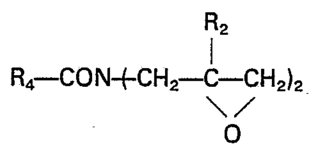

(II)

dans laquelle

$R_2$ est un atome d'hydrogène ou un groupe méthyle; et

$R_4$ est un groupe alcényle ayant 2 à 5 atomes de carbone.

3. Composé amidé N-glycidyl-substitué ayant la formule générale (III)

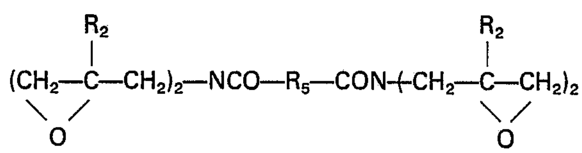

(III)

dans laquelle

$R_2$ est un atome d'hydrogène ou un groupe méthyle; et

$R_5$ est une liaison simple, un groupe alcoylène ayant 1 à 8 atomes de carbone ou un groupe alcénylène ayant 2 ou 3 atomes de carbone, ou un groupe phénylène.

4. Composé amidé N-glycidyl-substitué ayant la formule générale (IV)

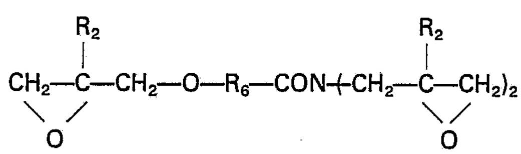

(IV)

dans laquelle

$R_2$ est un atome d'hydrogène ou un groupe méthyle; et

$R_6$ est un groupe alcoylène ayant 2 ou 3 atomes de carbone, ou un groupe arylène ayant 6 ou 7 atomes de carbone.

5. Composé choisi parmi le N,N-diglycidylacrylamide, le N,N-diglycidylméthacrylamide, le N,N-diglycidylcrotonamide, le N-méthyl-N-glycidylacrylamide, le N-méthyl-N-glycidylméthacrylamide, le N-n-butyl-N-glycidylacrylamide, le N-allyl-N-glycidylacétamide, le N-allyl-N-glycidylacrylamide, le N-benzyl-N-glycidylacrylamide ou le N-allyl-N-glycidylméthacrylamide.

6. Composé choisi parmi le N,N-di-2-méthylglycidylacrylamide, le N,N-di-2-méthylglycidylméthacrylamide, le N-butyl-N-2-méthylglycidylacrylamide, le N-allyl-N-2-méthylglycidylacétamide, le N-allyl-N-2-méthylglycidylacrylamide ou le N-allyl-N-2-méthylglycidylméthacrylamide.

7. Composé choisi parmi le N,N,N',N'-tétraglycidyloxamide, le N,N,N',N'-tétraglycidyladipamide, le N,N,N',N'-tétraglycidylfumaramide, le N,N,N',N'-tétraglycidylisophtalamide, le N,N,N',N'-tétraglycidyltéréphtalamide ou le N,N,N',N'-tétraglycidylphtalamide.

8. Composé choisi parmi le N,N,N',N'-tétra-2-méthylglycidyloxamide, le N,N,N',N'-tétra-2-méthylglycidylisophtalamide, ou le N,N,N',N'-tétra-2-méthylglycidyltéréphtalamide.

9. Composé choisi parmi le N,N-diglycidyl-α-glycidoxypropionamide, le N,N-diglycidyl-β-glycidoxypropionamide, le N,N-diglycidyl-p-glycidoxybenzamide ou le N,N-diglycidyl-o-glycidoxybenzamide.

10. Composé choisi parmi le N,N-di-2-méthylglycidyl-α-2-méthylglycidoxypropionamide, le N,N-di-2-méthylglycidyl-p-2-méthylglycidoxybenzamide ou le N,N-di-2-méthylglycidyl-o-2-méthylglycidoxybenzamide.